# EUROPEAN PATENT SPECIFICATION

(11) **EP 0 613 379 B1**
(45) Date of publication and mention of the grant of the patent: **12.08.1998**
(21) Application number: 92922888.0
(22) Date of filing: 10.11.1992
(51) Int. Cl.: A61K 51/04, A61K 121/00

(54) **THERAPEUTIC COMPOUNDS**
Therapeutische Mittel
COMPOSES THERAPEUTIQUES

(30) Priority: 12.11.1991 GB 9123947
(43) Date of publication of application: 07.09.1994
(73) Proprietor: IMPERIAL CANCER RESEARCH TECHNOLOGY LIMITED, London WC2A 3NL (GB)
(72) Inventor: EPENETOS, Agamemnon Antoniou ICRF Oncology Group, Hospital, DuCane Road, London W12 0HS (GB)
(74) Representative: Bassett, Richard Simon
(86) International application number: GB9202073
(87) International publication number: WO9309813

(56) References cited:
- EP-A- 0 174 853
- EP-A- 0 381 763
- EP-A- 0 490 434
- WO-A-89/12110
- WO-A-90/10448
- WO-A-91/04753
- WO-A-91/18012
- WO-A-92/02641

## Description

The present invention relates to therapeutic compounds useful in the treatment of neoplasms (ie cancers, tumours), viral diseases and other conditions.

It is known, at least on a theoretical basis, to administer antisense oligonucleotides (DNA or RNA) to inhibit the function *in vivo* of the corresponding sense nucleotides (DNA or RNA). Such antisense oligonucleotides are disclosed by Tullis in EP 92574 B1.

EP 0 490 434 describes labelled modified oligonucleotides useful for gene therapy.

WO 90/10448 describes covalent conjugates of a lipid and oligonucleotide.

WO 89/12110 describes a polynucleotide probe with a plurality of signalling moieties.

WO 91/04753 describes conjugates consisting of one or more antisense oligonucleotides bound to a ligand binding molecule which recognises a cell surface molecule.

We now provide improved compounds incorporating antisense oligonucleotides.

The invention provides a compound comprising a means to direct the compound to a nucleic acid within a cell and a radioactive moiety which, in use, destroys adjacent biological matter, particularly nucleic acid.

It is preferred that the means to direct the compound comprises an antisense oligonucleotide.

Antisense oligonucleotides are single-stranded nucleic acid, which can specifically bind to a complementary nucleic acid sequence. By binding to the appropriate target sequence, an RNA-RNA, a DNA-DNA, or RNA-DNA duplex is formed. These nucleic acids are often termed "antisense" because they are complementary to the sense or coding strand of the gene. Recently, formation of a triple helix has proven possible where the oligonucleotide is bound to a DNA duplex. It was found that oligonucleotides could recognise sequences in the major groove of the DNA double helix. A triple helix was formed thereby. This suggests that it is possible to synthesise sequence-specific molecules which specifically bind double-stranded DNA via recognition of major groove hydrogen binding sites.

By binding to the target nucleic acid, the above oligonucleotides can inhibit the function of the target nucleic acid. This could, for example, be a result of blocking the transcription, processing, poly(A) addition, replication, translation, or promoting inhibitory mechanisms of the cells, such as promoting RNA degradations.

The antisense oligonucleotide can be used to selectively suppress certain cellular functions. For example, in oncogenic transformed cells, oligonucleotides complementary to the oncogene suppress its expression. An antisense oligonucleotide has been shown to inhibit c-myc protein expression in a human promyelocytic leukaemia cell line, HL60, which over expresses the c-myc proto-oncogene. The antisense oligonucleotide used was complementary to regions of the c-myc mRNA.

Antisense oligonucleotides can also be used to inhibit replication and expression of nucleic acid foreign to the host cells. Antisense oligonucleotides are prepared in the laboratory and then introduced into cells, for example by microinjection or uptake from the cell culture medium into the cells, or they are expressed in cells after transfection with plasmids or retroviruses carrying an antisense gene. Antisense oligonucleotides were first discovered to inhibit viral replication or expression in cell culture for Rous sarcoma virus, vesicular stomatitis virus, herpes simplex virus type 1, simian virus and influenza virus. Since then, inhibition of mRNA translation by antisense oligonucleotides has been studied extensively in cell-free systems including rabbit reticulocyte lysates and wheat germ extracts. Inhibition of viral function by antisense oligonucleotides has been demonstrated *in vitro* using oligonucleotides which were complementary to the AIDS HIV retrovirus RNA (Goodchild, J. 1988 "Inhibition of Human Immunodeficiency Virus Replication by Antisense Oligedeoxynucleotides", *Proc. Natl. Acad. Sci. (USA)* **85**(15), 5507-11). The Goodchild study showed that oligonucleotides that were most effective were complementary to the poly(A) signal; also effective were those targeted at the 5' end of the RNA, particularly the cap and 5' untranslated region, next to the primer binding site and at the primer binding site. The cap, 5' untranslated region, and poly(A) signal lie within the sequence repeated at the ends of retrovirus RNA (R region) and the oligonucleotides complementary to these may bind twice to the RNA.

The antisense oligonucleotide may be any useful antisense oligonucleotide, for example an oligonucleotide complementary to DNA or RNA specifically forming part of a gene for the mutant *ras* protein, the mutant p53 protein, the BCR-ABL fused mRNA characteristic of the Philadelphia chromosome in chronic myeloid leukaemia (CML) and acute lymphocytic leukaemia (ALL), or an HIV (human immunodeficiency virus) protein, for example the HIV *gag*, *pol, env* or *sor* gene products. By "specifically", we mean that the complementary DNA or RNA does not normally appear in a normal (non-tumour or non-virally-infected) cell. Further targets for the antisense DNA/RNA include the HIV tRNA (Lys) primer binding site, mRNA splice donor or acceptor sites, the poly A region and the initiator codons of the HIV genes mentioned above.

Thus, in the case of CML, the oligonucleotide, GCTGAAGGGCTT^TTGAACTCTGCTTA hybridises to BCR exon 3/ABL and exon II junction sequences; and oligonucleotide GCTGAAGGGCTT^CTTCCTTATTGATG hybridises to BCR exon 2-ABL and exon II fusions. In the case of ALL, oligonucleotide GCTGAAGGGCTT^CTGCGTCTCCAT hybridises to the junction of BCR/ABL. In the above sequences, the circumflex denotes the junction between BCR and ABL exons.

Alternatively, the following sequences may be used:- CTGGTCTAACCAGAGAGACC (designated BB01); and GCAAGCTTTATTGAGGCTTA (designated BB02). A control could have the following sequence: CAGTCAGTCAGTCAGTCAGT (designated BB03). BB01 is complementary to the cap or initiator codon of HIV and BB02 is complementary to the poly(A) signal of the HIV genomic RNA. The cap and poly(A) signal lie within the sequence repeated at the ends of the HIV RNA (R region). BB03 is a 20-mer not complementary to the HIV RNA. BB03 has been tested and shown to be inactive (Goodchild, J. *supra*).

Further HIV oligonucleotides include: CTGCTAGAGATddT; TGCTAGAGATTTTCCACAC; TTCAAGTCCCTGTTCGGGCGCCAAA; GCGTACTCACCAGTCGCCGC; CTGCTAGAGATTAA; ACACCCAATTCTGAAAATGG; and equivalents thereof. Alternatively, the oligonucleotide can target HIV nucleotide sequences which code for a protease necessary for proper viral assembly. Oligodeoxynucleotides blocked at the 3' end by ddT, the isourea group or other chain terminators may prove to be more effective inhibitors. In general, any highly conserved region of the HIV genome which encodes information necessary for viral replication or gene expression (eg protein synthesis) is a potential target for complementary oligodeoxynucleotides. Further, the oligonucleotide can be complementary to the viral mRNA, one strand of an integrated or unintegrated proviral DNA, a DNA-RNA, or RNA-RNA duplexes.

Similarly, the antisense oligonucleotide conjugate can be used to inhibit replication or expression of other viruses, for example, herpes viruses in the treatment of herpes. Additionally, in the case of DNA viruses, the oligonucleotides can be complementary to the genomic DNA.

A further embodiment of the invention provides a nucleic acid, preferably DNA, directed to a nucleic acid within a cell wherein the DNA is capable of homologous recombination with the said nucleic acid (DNA) within a cell. In this embodiment it is preferred that the DNA of the invention contains between 100 nucleotides and 50 000 nucleotides, more preferably between 500 and 10 000, or between 1000 and 5000; it is further preferred that the DNA is double-stranded.

The DNA of this embodiment of the invention may be designed to recombine homologously with specific DNA sequences within the cells to be targeted. In particular, cells containing gross chromosomal modifications which for example alter say between 100 bp and 100 kb sections of a chromosome, or have insertions or deletions of between 100 bp and 100 kb, or are translocations between or within chromosomes provide candidate targets for the DNA.

Thus, a cell containing such chromosome modifications may be targeted. An example of such cells are chronic myeloid leukaemia cells which contain the Philadelphia chromosome (as disclosed above) characterised by a translocation which juxtaposes parts of the *bcr* and parts of the *abl* genes. DNA of the invention containing between 1000 and 5000 bp of the *bcr* gene fused to between 1000 and 5000 bp of the *abl* gene in such a way that this fusion corresponded to the sequence cf the *bcr-abl* fusion on the Philadelphia chromosome may be useful in treating CML.

The foregoing approach may be used to treat diseases other than cancers and viral infections, and may be applied to the treatment of sepsis, as described below.

### TNF-Induced Diseases/Symptoms

Examples of antisense oligonucleotides that can be used for preventing or suppressing TNF-induced diseases for example sepsis, are those complementary to TNF DNA or TNF RNA. For example, oligonucleotides complementary to the following can be used: sequences around the 5' end of the TNF messenger RNA; sequences at the beginning of and within the mRNA region coding for the transmembrane domain of the TNF protein; and sequences within the coding region of the 17kD molecule. Examples of the specific oligonucleotide sequences complementary to the above mRNA regions are:
5'TCTCCCTCTTAGCTGGTCCTCTGC3';
5'CATGCTTTCAGTGCTCATGGTGTCCTTTC3';
5'GATCAGGAAGGAGAAGAGGCTGAGGAACAA3';
5'CTCAGCTTGAGGGTTTGC3'; and
5'TTCGTCCTCCTCACAGGGC3'.

It will be apparent to those skilled in the art that the oligonucleotide used in the treatment of the above diseases, and other applications, can be either an oligodeoxynucleotide or an oligoribonucleotide. Among other factors, the choice will be dependent on the case of synthesis, the efficacy, and the relative stability and special advantages of the oligonucleotides in a particular system. Further, the oligonucleotides can be complementary to either DNA or RNA. It can also bind to either or both single-stranded or double-stranded nucleic acid. The DNA or RNA can be indigenous (cellular) to the cell in question or it can be foreign nucleic acid found in the host cells. The DNA can be cellular or foreign infectious DNA, eg those of viruses, bacteria, yeast, fungi and other parasites. The RNA can be genomic RNA or messenger RNA, for example retroviral genomic RNA or foreign or cellular mRNA. Where the oligonucleotide is complementary to and bound to the genomic DNA or RNA, it inhibits or prevents the nucleic acid from being replicated. By interfering with or inhibiting the replication of the nucleic acid, the oligonucleotide interferes with or inhibits downstream expression of the DNA or RNA in protein synthesis. Where the oligonucleotide is complementary to the messenger RNA it interferes with or inhibits the mRNA from being expressed in protein synthesis.

Of course, the oligonucleotides may be "modified oligonucleotides".

By "modified oligonucleotides" we mean that they may contain phosphorothioate, methylphosphonate or other phosphoramidite internucleosidic linkages as well as, or instead of the usual phosphodiester linkages. Such internucleosidic linkages are less susceptible to nucleolytic degradation, or may confer on the antisense oligonucleotide other preferred pharmacokinetic properties. A further modification that can be made instead of or in addition to the aforementioned modifications is the addition of a component capable of intercalating into the target nucleic acid, and thus stabilising the resultant (antisense oligonucleotide):(target nucleic acid) hybrid. The intercalating component is preferably acridine.

An enhanced inhibitory effect can be obtained by rendering the antisense oligonucleotide, or other means to direct the compound to a nucleic acid within the cell, radioactive.

The radioactive moiety may comprise phosphorus-32. However, more preferably it is iodine-125, iodine-131, indium-111, rhenium-186, rhenium-188 or yttrium-90, or any other isotope which emits enough energy to destroy neighbouring cells, organelles or nucleic acid. Preferably, the isotopes and density of radioactive atoms in the compound of the invention are such that a dose of more than 4000 cGy (preferably at least 6000, 8000 or 10000 cGy) is delivered to the cell and its organelles, particularly the nucleus.

The radioactive atom(s) may be incorporated in the compound of the invention in known ways. For example, the first portion may be biosynthesized or may be synthesized by *in vitro* synthesis using in each case suitable radioactive nucleotides, nucleosides or bases, for example. A pre-formed oligonucleotide may be labelled with ^{32p} using T4 polynucleotide Kinase and γ-[^{32p}]ATP.

EDTA or another chelating agent may be attached to a 5'-phosphate group (FEBS Letters (1984), **172**, 43-46) and used to attach ¹¹¹In or ⁹⁰Y, for example. Tyrosine can be esterified to the 3'-hydroxyl group and labelled with ¹²⁵I or ¹³¹I.

The compound may additionally comprise a portion capable of targeting the compound to cells generally or to a desired cell type.

By "capable of" we mean capable of targeting the compound as said when the said targeting portion is part of the compound of the invention.

The targeting portion may specifically bind to a cell-type-specific entity or may be specifically taken up by the specific cell type which is the intended target.

The entity recognised may be characteristic of cells in general, so that the antisense oligonucleotide is simply taken up into cells and is therefore exposed less to extracellular nucleases, for example. The specificity of the compound is thus derived solely from the antisense oligonucleotide.

Alternatively, the entity which is recognised may be a suitable entity which is specifically expressed by tumour cells, virally-infected cells, pathogenic microorganisms, cells introduced as part of gene therapy or even specific normal cells of the body into which, for whatever reason, one wishes to introduce the antisense oligonucleotide, but which entity is not expressed, or at least not with such frequency, in cells into which one does not wish to introduce the oligonucleotide. The entity which is recognised will often be an antigen. Examples of antigens include those listed in Table 1 below. A non-specific antigen is the transferrin receptor, to which antibodies may be raised, as taught in EP 226 419. Monoclonal antibodies which will bind specifically to many of these antigens are already known (for example those given in the Table) but in any case, with today's techniques in relation to monoclonal antibody technology, antibodies can be prepared to most antigens. The antigen-specific portion may be an entire antibody (usually, for convenience and specificity, a monoclonal antibody), a part or parts thereof (for example an F_{ab} fragment, F(ab')₂, dab or "minimum recognition unit") or a synthetic antibody or part thereof. A compound comprising only part of an antibody may be advantageous by virtue of being less likely to undergo non-specific binding due to the F_{c} part. Suitable monoclonal antibodies to selected antigens may be prepared by known techniques, for example those disclosed in "Monoclonal Antibodies: A manual of techniques", H. Zola (CRC Press, 1988) and in "Monoclonal Hybridoma Antibodies: Techniques and Applications", J.G.R. Hurrell (CRC Press, 1982). All references mentioned in this specification are incorporated herein by reference. Bispecific antibodies may be prepared by cell fusion, by reassociation of monovalent fragments or by chemical cross-linking of whole antibodies, with one part of the resulting bispecific antibody being directed to the cell-specific antigen and the other to the oligonucleotide. The bispecific antibody can be administered bound to the oligonucleotide or it can be administered first, followed by the oligonucleotide. The former is preferred. Methods for preparing bispecific antibodies are disclosed in Corvalan *et al* (1987) *Cancer Immunol. Immunother.* **24**, 127-132 and 133-137 and 138-143. Bispecific antibodies, chimaeric antibodies and single chain antibodies are discussed generally by Williams in *Tibtech*, February 1988, Vol. 6, 36-42, Neuberger *et al* (*8th International Biotechnology Symposium*, 1988, Part 2, 792-799) and Tan and Morrison (*Adv. Drug Delivery Reviews* **2**, (1988), 129-142). Suitably prepared non-human antibodies can be "humanized" in known ways, for example by inserting the CDR regions of mouse antibodies into the framework of human antibodies. IgG class antibodies are preferred.

**Table 1**

| 1. Tumour Associated Antigens | | |
|---|---|---|
| Antigen | Antibody | Existing Uses |
| Carcino-embryonic Antigen | {C46 (Amersham) {85A12 (Unipath) | Imaging & Therapy of colon/rectum tumours. |
| Placental Alkaline Phosphatase | H17E2 (ICRF, Travers & Bodmer | Imaging & Therapy of testicular and ovarian cancers. |
| Pan carcinoma | NR-LU-10 (NeoRx Corporation) | Imaging & Therapy of various carcinomas incl. small cell lung cancer. |
| Polymorphic Epithelial | HMFG1 (Taylor- | Imaging & Therapy of ovarian cancer, |
| Mucin (Human milk fat globule) | Papadimitriou, ICRF) | pleural effusions. |
| β-human Chorionic Gonadotropin | W14 | Targeting of enzyme (CPG2) to human xenograft choriocarcinoma in nude mice. (Searle *et al* (1981) *Br. J. Cancer* **44**, 137-144). |
| a Carbohydrate on Human Carcinomas | L6 (IgG2a)¹ | Targeting of alkaline phosphatase. (Senter *et al* (1988) *P.N.A.S.* **85**, 4842-4846. |
| CD20 Antigen on B Lymphoma (normal and and neoplastic) | 1F5 (IgG2a)² | Targeting of alkaline phosphatase. (Senter *et al* (1988) *P.N.A.S.* **85**, 4842-4846. |
| Other antigens include alphafoetoprotein, Ca-125 and prostate specific antigen. | | |

| 2. Immune Cell Antigens | | |
|---|---|---|
| Pan T Lymphocyte Surface Antigen (CD3) | OKT-3 (Ortho) | As anti-rejection therapy for kidney transplants. |
| B-lymphocyte Surface Antigen (CD22) | RFB4 (Janossy, Royal Free Hospital) | Immunotoxin therapy of B cell lymphoma. |
| Pan T lymphocyte Surface Antigen (CD5) | H65 (Bodmer, Knowles ICRF, Licensed to Xoma Corp., USA) | Immunotoxin treatment of Acute Graft venus Host disease, Rheumatoid Arthritis. |

| 3. Infectious Agent-Related Antigens | | |
|---|---|---|
| Mumps virus-related | Anti-mumps polyclonal antibody | Antibody conjugated to Diphtheria toxin for treatment of mumps. |
| Hepatitis B Surface Antigen | Anti HBs Ag | Immunotoxin against Hepatoma. |

| | | |
|---|---|---|
| ¹Hellström *et al* (1986) *Cancer Res*. **46**, 3917-3923 | | |
| ²Clarke *et al* (1985) *P.N.A.S.* **82**, 1766-1770 | | |

If applied to the treatment of CML or ALL, the ligand binding molecules Can be monoclonal antibodies against leukaemiaassociated antigens. Examples of these are: anti-CALLA (Common acute lymphoblastic leukaemia-associated antigen), J5, BA-3, RFB-1, BA-2, SJ-9A4 Du-ALL-1, anti-3-3, anti-3-40, SN1 and CALL2, described in Foon, K.A. *et al* 1986 *Blood* **68**(1), 1-31, "Review: Immunologic Classification of Leukemia and Lymphoma". The ligand binding molecules can also be antibodies that identify myeloid cell surface antigens, or antibodies that are reactive with B or T lymphocytes, respectively. Examples of such antibodies are those which identify human myeloid cell surface antigens or those which are reactive with human B or T lymphocytes as described in Foon, K.A. *Id.* Additional examples are antibodies B43, CD22 and CD19 which are reactive with B lymphocytes can also be used.

Alternatively, the entity which is recognised may or may not be antigenic but can be recognised and selectively bound to in some other way. For example, it may be a characteristic cell surface receptor such as the receptor for melanocyte-stimulating hormone (MSH) which is expressed in high numbers in melanoma cells. The cell-specific portion may then be a compound or part thereof which specifically binds to the entity in a non-immune sense, for example as a substrate or analogue thereof for a cell-surface enzyme or as a messenger. In the case of melanoma cells, the cell-specific portion may be MSH itself or a part thereof which binds to the MSH receptor. Such MSH peptides are disclosed in, for example, Al-Obeidi *et al* (1980) J. *Med. Chem.* **32**, 174. The specificity may be indirect: a first cell-specific antibody may be administered, followed by a compound of the invention directed against the first antibody. Preferably, the entity which is recognised is not secreted to any relevant extent into body fluids, since otherwise the requisite specificity may not be achieved.

The targeting portion of the compound of this embodiment of the invention may be linked to the remainder of the compound by any of the conventional ways of linking compounds, for example by disulphide, amide or thioether bonds, such as those generally described in Goodchild, *supra* or in Connolly (1985) *Nucl. Acids Res.* **13**(12), 4485-4502 or in PCT/US85/03312. A thiol group can be introduced at the 5'-end of an aminofunctionalised oligonucleotide (ref: *Nucleic Acids Res*. (1991) **19**, 4561). This group can be used to attach the oligonucleotide to a protein, such as a monoclonal antibody or growth factor, using standard heterobiofunctional protein cross-linking reagents such as m-maleimidobenzoylN-hydroxysuccinimide ester (MBS). These reagents usually link between a thiol group in one protein and the terminal amino group in a lysine residue in the other protein. Preferably, the linkage is cleavable in lysosomes by lysosomal enzymes or by the acidic environment to liberate the antisense oligonucleotide.

The compounds of the invention may be administered in any suitable way, usually parenterally, for example intravenously, intraperitoneally or, preferably (for bladder cancers), intra-vesically (ie into the bladder), in standard sterile, non-pyrogenic formulations of diluents and carriers, for example isotonic saline (when administered intravenously). If needed, because the compound of the invention may be immunogenic, cyclosporin or some other immunosuppressant can be administered to provide a longer period for treatment but usually this will not be necessary.

Particular tumours suitable for treatment in accordance with either aspect of the invention include cancers of the uterine cervix, head, neck, brain gliomas, breast, colon, oesophagus, stomach, liver, pancreas and metastatic forms of any of these.

If desired, the antisense oligonucleotide can be conjugated with hydrophobic derivatives as taught in FR 2 649 321 to protect it from nucleases and to improve transport across cell membranes. The hydrophobic moiety may be cholesterol as taught by Zon in "*Oligodeoxynucleotides: Antisense Inhibitors* *of Gene Expression*", pp 234-247, J.S. Cohen (Ed), CRC Press, Boca Raton, FL, 1989.

Conjugation of the oligonucleotides to poly-L-lysine may also enhance delivery of the said oligonucleotides to the cell as disclosed by Stevenson and Iversen (1989) *J. Gen. Virol.* **70**, 2673-2682, and by LeMaitre *et al* (1987) *Proc. Natl. Acad. Sci. USA* **84**, 648-652.

In a similar fashion polyamines conjugated to phosphorothioate oligonucleotides enhance their cellular uptake as taught in US 5 138 045.

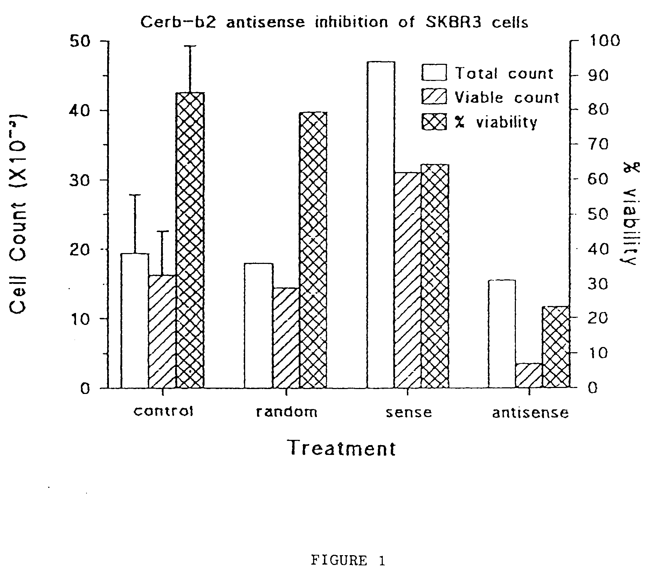

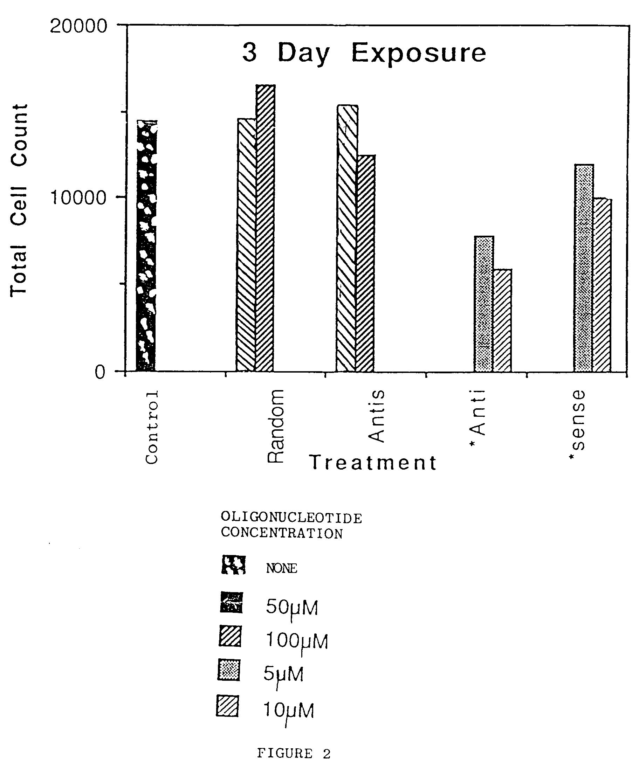

The invention will now be described in detail by reference to the following Examples and Figures in which Figure 1 shows the effect of non-radioactive random, c-*erb*-B2 sense and c-*erb*-B2 antisense oligonucleotides on the viability of SKBR3 cells and Figure 2 shows the effect of ¹²⁵I-labelled c-*erb*-B2 antisense and c-*erb*-B2 sense oligonucleotides.

### EXAMPLE 1

The chosen oligonucleotide (see below) is labelled at the 3' end by esterifying thereto a tyrosine group and labelling it with ¹²⁵I by known methods.

In order to form a conjugate, oligonucleotides with a reactive sulphydryl group were synthesized. In the preferred embodiment, the oligonucleotides with reactive sulphydryl groups were designated BB04, BB05, and BB06, each with a sequence corresponding to BB01, BB02, and BB03 respectively (see above). The following reaction conditions were used:

| BB01 20-MER | |
|---|---|
| CTGGTCTAACCAGAGAGACC | |
| MW AMMONIUM SALT | 6417.65 |
| MOLAR EXTINCTION AT 260nm | 195800 |
| MICROGRAMS PER OD260nm | 32.78 |
| PICOMOLES PER OD260nm | 5107.25 |
| BASE COMPOSITION: ACGT | 6653 |
| Td (blot) 0.1M Na+ | 62 |
| Tm @ 0.1M Na+, 0.000001M Probe | 58 |

| BB02 20-MER | |
|---|---|
| GCAAGCTTTATTGAGGCTTA | |
| MW AMMONIUM SALT | 6453.67 |
| MOLAR EXTINCTION AT 260nm | 193700 |
| MICROGRAMS PER OD260nm | 33.32 |
| PICOMOLES PER OD260nm | 5162.62 |
| BASE COMPOSITION: ACGT | 5357 |
| Td (blot) 0.1M Na+ | 56 |
| Tm @ 0.1M Na+, 0.000001M Probe | 60 |

| BB03 20-MER | |
|---|---|
| CAGTCAGTCAGTCAGTCAGT | |
| MW AMMONIUM SALT | 6423.65 |
| MOLAR EXTINCTION AT 260nm | 196900 |
| MICROGRAMS PER OD260nm | 32.62 |
| PICOMOLES PER OD260nm | 5078.72 |
| BASE COMPOSITION: ACGT | 5555 |
| Td (blot) 0.1M Na+ | 60 |
| Tm @ 0.1M Na+, 0.000001M Probe | 55 |

The sulphydryl analogues were as follows:

| BB04 21-MER | |
|---|---|
| XCTGGTCTAACCAGAGAGACC | |
| MW AMMONIUM SALT | 6417.65 |
| MOLAR EXTINCTION AT 260nm | 220400 |
| MICROGRAMS PER OD260nm | 29.12 |
| PICOMOLES PER OD260nm | 4537.21 |
| BASE COMPOSITION: ACGT | 6653 |
| MIXED BASES: YRNMKSWHBVDXZ | 0000000000010 |
| X=ANTITRANSFERRIN RECEPTOR ANTIBODY, | |
| Td (blot) 0.1M Na+ | 63 |
| Tm @ 0.1M Na+, 0.000001M Probe | 58 |

| BB05 21-MER | |
|---|---|
| XGCAAGCTTTATTGAGGCTTA | |
| MW AMMONIUM SALT | 6453.67 |
| MOLAR EXTINCTION AT 260nm | 217600 |
| MICROGRAMS PER OD260nm | 29.66 |
| PICOMOLES PER OD260nm | 4595.59 |
| BASE COMPOSITION: ACGT | 5357 |
| MIXED BASES: YRNMKSWHBVDXZ | 0000000000010 |
| X=ANTITRANSFERRIN RECEPTOR ANTIBODY, | |
| Td (blot) 0.1M Na+ | 57 |
| Tm @ 0.1M Na+, 0.000001M Probe | 60 |

| BB06 21-MER | |
|---|---|
| XCAGTCAGTCAGTCAGTCAGT | |
| MW AMMONIUM SALT | 6423.65 |
| MOLAR EXTINCTION AT 260nm | 215000 |
| MICROGRAMS PER OD260nm | 29.88 |
| PICOMOLES PER OD260nm | 4651.16 |
| BASE COMPOSITION: ACGT | 5555 |
| MIXED BASES: YRNMKSWHBVDXZ | 0000000000010 |
| X=ANTITRANSFERRIN RECEPTOR ANTIBODY, | |
| Td (blot) 0.1M Na+ | 61 |
| Tm @ 0.1M Na+, 0.000001M Probe | 55 |

The oligonucleotides had the following generalised structure (the sulphydryl group was denoted as X above:

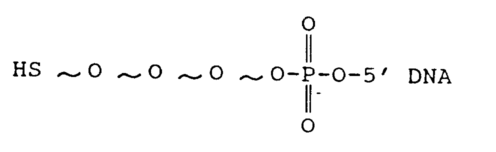

At the same time, a sulphydryl group was added to an anti-HIV antigen antibody. The oligonucleotide was covalently linked to the MAb through a disulphide exchange reaction between the sulphydryl groups of the two compounds. This reaction is described below.

### Addition of Sulphydryl Group on the Antibody

The addition of a thiol group or an activated disulphide group to an immunoglobulin is known in relation to the synthesis of immunotoxins (US Patent No 4,340,535). The procedures disclosed therein regarding addition of the thiol group to the antibody are incorporated hereby.

Ellman's reagent is added to a solution of antibody in 40 mM phosphate buffer containing 1 mM EDTA. The final concentration of MAb is 3.2 mg/ml, and that of Ellman's reagent is 1 mM. Final pH of the mixture is 8. The reaction is allowed to proceed for 30 minutes at room temperature. At the end of 30 minutes, the reaction mixture is cooled in an ice bucket to 4°C and a ten fold excess of 2-iminothiolane reagent is added. The reaction mixture is allowed to continue at 4°C overnight. At the end of the reaction, the excess reagents are separated on a 1.5 x 15 cm column of Sephadex G-25 equilibrated with 40 mM sodium phosphate, pH 7.6, containing 0.2 M NaCl and 1 mM EDTA.

### Disulphide Linkage of the Oligonucleotide with the Antibody

The derivatised antibody is then covalently linked, in a disulphide exchange, to the sulphydryl group on the oligonucleotide. This linkage was achieved by incubating the two components (MAb-IT-TNB at 4 nM and oligonucleotide at 100 nM, final concentration) overnight at 4°C. The sample will turn yellow, indicating that the TNB group is being released and the desired product is being formed.

The sample is then passed through a 2.5 x 24 cm column of Sephadex G-25 resin equilibrated with 40 mM sodium phosphate, pH 7.6, containing 0.2 M NaCl. The separation profile of a standard mixture of proteins may be compared to MAb.

### EXAMPLE 2

### Synthesis of Oligonucleotide Antibody Conjugates Through Thioether Bonding

In general, the procedure for forming the oligonucleotide antibody conjugate consists of reacting antibody, either polyclonal or monoclonal, having a free amino group with a maleimide-active ester in a suitably buffered solution. Preferably, the maleimide-active ester is present in about a two-fold molar excess over antibody, and the pH of the solution is slightly alkaline to maintain the antibody's amino group in an unprotonated state. The reaction of antibody with the thioether crosslinker can be followed by monitoring the absorbance of the solution at a wavelength of about 405 nm. An increase in absorbance at this wavelength is the result of the dianion leaving group, HNSA, and the reaction of antibody amines to form stable amide bonds. Because hydrolysis of the crosslinker's active ester is slow relative to aminolysis, most of the leaving groups' absorbance is due to amide bond formation. The reaction of antibody with the crosslinker is for a time sufficient to introduce about 0.5-3 crosslinker molecules per antibody molecule. Next, the derivatised antibody is separated from the crosslinker, using any number of standard biochemical separation techniques. Preferably the separation procedure will be accomplished using a gel filtration column, and more preferably Sephadex G-25 (Regd. T.M. Pharmacia Corp.) will be employed. The column is pre-equilibrated with a chromatographically compatible aqueous buffered solution. The isolated derivatised antibody can then be reacted with the oligonucleotide having a sulphydryl group as described below.

Oligonucleotide having a free sulphydryl group can be directly reacted with the derivatised antibody in an aqueous buffered solution compatible with the reaction. The oligonucleotide and antibody concentrations, and the duration of the reaction may vary depending on the number of oligonucleotide molecules sought to be bound to antibody. The reaction is preferably run at 4°C overnight.

### Synthesis of the Preferred Oligonucleotide-Antibody Conjugates

More specifically, the synthesis of oligonucleotide-antibody conjugate is carried out as follows.

The monoclonal antibody is reacted with the heterobifunctional crosslinker, mal-sac-spacer-glut-HNSA- as follows. 10 mg/ml of MAb is reacted with a two-fold molar excess of the thioether crosslinker in 0.1 M sodium phosphate. pH 8, for about 25 minutes at room temperature. The progress of the reaction is followed by measuring the absorbance at 406 nm. At the end of 25 minutes the absorbance may have increased to 0.57, and the derivatised antibody is separated from the reaction mixture by gel filtration using a Sephadex G-25 column (2.5 x 17 cm) in 40 mM sodium phosphate buffer, pH 6, containing 200 mM NaCl. This material is reacted, as described below, with the oligonucleotide having a reactive sulphydryl group.

The oligonucleotide with reactive sulphydryl group was combined with derivatised antibody in a 1:2 molar ratio (antibody:oligonucleotide with sulphydryl group). The solution is concentrated using an Amicon stirred ultra-filtration device. The buffer employed is 40 mM sodium phosphate, pH 7.6, containing 200 mM NaCl. The reaction is allowed to proceed overnight at 4°C, and the sample is then chromatographed over GF 250 gel filtration column (PBS pH 7.6). The fractions collected are run on 6.5% SDS-PAGE and conjugates should be observed having molecular weight greater than the unconjugated antibody.

Alternatively, to maximise the conjugation, the oligonucleotide with reactive sulphydryl group is combined with derivatised antibody in a 1:25 molar ratio (antibody:oligonucleotides with sulphydryl group). The oligonucleotide and antibody concentrations may vary depending on the number of oligonucleotides bound to the antibody. Additionally, after the reaction has proceeded overnight at 4°C, the sample can be chromatographed over a Sepharose (Regd. T.M.) S-300 column (2.2 x 80 cm) in 40 mM sodium phosphate, pH 6.5, containing 200 mM NaCl. This step removed any unreacted oligonucleotides with the sulphydryl group.

Fractions containing either free antibody, if any, or oligonucleotide antibody conjugate can be identified using a suitable analytical technique, such as sodium dodecyl sulphate polyacrylamide gel electrophoresis. The oligonucleotide antibody conjugates so isolated can, if desired, be concentrated by any suitable technique known in the art followed by sterilisation. The latter is readily achieved by passing the oligonucleotide antibody conjugate through an 0.2 micron filter.

The above conjugates may be as efficacious as conjugates formed by disulphide linkages, and the methods of use of both types of conjugates are similar.

### EXAMPLE 3

### Selective Killing of SKBR3 Cells by Radiolabelled c-erb-B2 Oligonucleotides

The following oligonucleotides were synthesised containing phosphorothioate linkages:

### (a) Antisense

### 5'-CACAAGGCCGCCAGCTC-3'

This sequence is complementary to the non-transcribed sequence at the 5' end of the gene.

### (b) Sense

### 5'-GAGCTGGCGGCCTTGTG-3'

This sequence is complementary to the transcribed sequence at the 5' end of the gene.

### (c) Random

### 5-CTGGCACGACGCACACC-3'

This is a random sequence, but has the same base-composition as the antisense oligonucleotide.

The oligonucleotides were radiolabelled with iodine-125 using the Iodogen method of Mieczyslaw A. *et al* (1988) *Analytical Biochemistry* **172**, 356-359. Typically, ¹²⁵I-labelled oligonucleotides had a specific activity of 0.7MBq mg⁻¹.

SKBR3 cells in 9 cm culture plates were grown in Dulbecco's modified MEM containing 10% foetal calf serum supplement. Subconfluent cells were detached using trypsin/versene, and plated out at a density of 1 x 10⁴ cells per microtitre well (0.5 ml) in 24 well plates. Oligonucleotides (antisense, sense or random) were added at 50 µM or 5 µM. Cells were then grown at 37°C in an incubator with an atmosphere of 10% CO₂ in air. After 3 days cells were harvested using trypsin/versene, at which time cells were counted and their viability assessed by the trypan blue exclusion test.

Figure 1 shows the cell count when unlabelled random, sense and antisense c-*erb*-B2 oligonucleotides are used to inhibit SKBR3 cells. The control shows the effect of the same treatment but with no oligonucleotide present. The antisense oligonucleotide is considerably more effective in reducing cell viability than any of the controls.

Figure 2 shows that the radioactive antisense oligonucleotide (*Anti) is considerably more potent than the unlabelled random and antisense oligonucleotide or the labelled sense oligonucleotides (*Sense). In Figure 2 all cells were >96% viable immediately following treatment. The concentrations given are those of the oligonucleotides, and the results indicate that the ¹²⁵I-labelled antisense oligonucleotide appears to have a toxic effect on the SKBR3 cells at concentrations 1/10th that of the unlabelled antisense oligonucleotide.

## Claims

1. A compound comprising an antisense oligonucleotide and a radioactive moiety which, in use, destroys adjacent biological matter wherein the radioactive moiety comprises indium-111, rhenium-188 or phosphorus-32.

2. A compound comprising an antisense oligonucleotide complementary to DNA or RNA specifically forming part of a gene for the mutant *ras* protein, the mutant p53 protein, mutant *myc* protein, the BCR-ABL protein or an HIV protein and a radioactive moiety which, in use, destroys adjacent biological matter.

3. A compound comprising a nucleic acid molecule capable of recombining homologously with a target nucleic acid sequence in the nucleus and a radioactive moiety which, in use, destroys adjacent biological matter.

4. A compound according to Claim 2 or 3 wherein the radioactive moiety comprises iodine-125, iodine-131, phosphorus-32, rhenium-186, rhenium-188 or yttrium-90.

5. A compound comprising an antisense oligonucleotide and a radioactive moiety which, in use, destroys adjacent biological matter additionally comprising a targeting portion to target the compound to cells generally wherein if the targeting portion is a lipid the radioactive moiety does not contain iodine-125 or phosphorous-32.

6. A compound comprising an antisense oligonucleotide and a radioactive moiety which, in use, destroys adjacent biological matter additionally comprising a targeting portion to target the compound to a desired cell type.

7. A compound according to Claim 3 additionally comprising a targeting portion to target the compound to cells generally or to a desired cell type.

8. A compound according to Claim 6 or 7 wherein the targeting portion is a monoclonal antibody or part thereof for a tumour-cell-specific or virally-infected-cell-specific entity.

9. A pharmaceutical composition comprising a compound according to any of the preceding claims and a pharmaceutical carrier.

10. A compound according to any one of claims 1 to 8 for use in medicine.

11. Use of a compound according to any one of Claims 1 to 8 in the manufacture of a medicament for treating a mammal having biological matter to be destroyed.

## Patentansprüche

1. Verbindung mit einem Antisense-Oligonucleotid und einer radioaktiven Einheit, die bei Verwendung benachbartes biologisches Material zerstört, wobei die radioaktive Einheit Indium-111, Rhenium-188 oder Phosphor-32 umfaßt.

2. Verbindung mit einem Antisense-Oligonucleotid, das komplementär zu DNA oder RNA ist, die speziell einen Teil eines Gens für das mutierte ras-Protein, das mutierte p53-Protein, das mutierte myc-Protein, das BCR-ABL-Protein oder ein HIV-Protein bildet, und einer radioaktiven Einheit, die bei Verwendung benachbartes biologisches Material zerstört.

3. Verbindung mit einem Nucleinsäuremolekül mit der Fähigkeit zur homologen Rekombination mit einer Target-nucleinsäuresequenz im Kern und einer radioaktiven Einheit, die bei Verwendung benachbartes biologisches Material zerstört.

4. Verbindung nach Anspruch 2 oder 3, wobei die radioaktive Einheit Iod-125, Iod-131, Phosphor-32, Rhenium-186, Rhenium-188 oder Yttrium-90 umfaßt.

5. Verbindung mit einem Antisense-Oligonucleotid und einer radioaktiven Einheit, die bei Verwendung benachbartes biologisches Material zerstört, wobei die Verbindung darüber hinaus einen Targetteil, so daß die Verbindung zielgerichtet allgemein auf Zellen gesteuert werden kann, enthält, wobei die radioaktive Einheit im Falle, daß der Targetteil ein Lipid ist, nicht Iod-125 oder Phosphor-32 enthält.

6. Verbindung mit einem Antisense-Oligonucleotid und einer radioaktiven Einheit, die bei Verwendung benachbartes biologisches Material zerstört, wobei die Verbindung darüber hinaus einen Targetteil, um die Verbindung zielgerichtet auf einen gewünschten Zelltyp zu steuern, enthält.

7. Verbindung nach Anspruch 3, die darüber hinaus einen Targetteil enthält, um die Verbindung zielgerichtet allgemein auf Zellen oder einen gewünschten Zelltyp zu steuern.

8. Verbindung nach Anspruch 6 oder 7, wobei der Targetteil ein monoklonaler Antikörper oder ein Teil hiervon für eine tumorzellspezifische Einheit oder für eine für durch Viren infizierte Zellen spezifische Einheit ist.

9. Arzneimittelzubereitung, die eine Verbindung nach einem der vorhergehenden Ansprüche und einen pharmazeutischen Träger enthält.

10. Verbindung nach einem der Ansprüche 1 bis 8 zur Verwendung in der Medizin.

11. Verwendung einer Verbindung nach einem der Ansprüche 1 bis 8 zur Herstellung eines Medikaments zur Behandlung eines Säugetiers, das zu zerstörendes biologisches Material besitzt.

## Revendications

1. Composé comprenant un oligonucléotide antisens et un fragment radioactif qui, lors de son utilisation, détruit la matière biologique adjacente, dans lequel le fragment radioactif comprend de l'indium-111, du rhénium-188 ou du phosphore-32.

2. Composé comprenant un oligonucléotide antisens complémentaire d'ADN ou d'ARN faisant spécifiquement partie d'un gène de la protéine *ras* mutante, de la protéine p53 mutante, de la protéine *myc* mutante, de la protéine BCR-ABL ou d'une protéine du VIH, et un fragment radioactif qui, lors de son utilisation, détruit la matière biologique adjacente,

3. Composé comprenant une molécule d'acide nucléique capable de recombinaison homologue avec une séquence d'acide nucléique cible dans le noyau et un fragment radioactif qui, lors de son utilisation, détruit la matière biologique adjacente.

4. Composé selon la revendication 2 ou 3 dans lequel le fragment radioactif comprend de l'iode-125, de l'iode-131, du phosphore-32, du rhénium-186, du rhénium-188 ou de l'yttrium-90.

5. Composé comprenant un oligonucléotide antisens et un fragment radioactif qui, lors de son utilisation, détruit la matière biologique adjacente, comprenant en outre une partie de ciblage destinée à diriger le compose vers des cellules en général, dans lequel, lorsque la partie de ciblage est un lipide, le fragment radioactif ne contient pas d'iode-125 ou de phosphore-32.

6. Composé comprenant un oligonucléotide antisens et un fragment radioactif qui, lors de son utilisation, détruit la matière biologique adjacente, comprenant en outre une partie de ciblage destinée à diriger le composé vers un type cellulaire désiré.

7. Composé selon la revendication 3 comprenant en outre une partie de ciblage destinée à diriger le composé vers des cellules en général ou vers un type cellulaire désiré.

8. Composé selon la revendication 6 ou 7, dans lequel la partie de ciblage est un anticorps monoclonal ou une partie de celui-ci pour une entité spécifique de cellules tumorales ou spécifique de cellules infectées par un virus.

9. Composition pharmaceutique comprenant un composé selon l'une quelconque des revendications précédentes et un véhicule pharmaceutique.

10. Composé selon l'une quelconque des revendications 1 à 8 pour une utilisation en médecine.

11. Utilisation d'un composé selon l'une quelconque des revendications 1 à 8 dans la fabrication d'un médicament pour le traitement d'un mammifère chez lequel de la matière biologique doit être détruite.


SEQUENCE LISTING

(1) GENERAL INFORMATION:

(i) APPLICANT: Epenetos, Agamemnon A

(ii) TITLE OF INVENTION: Therapeutic Compounds

(iii) NUMBER OF SEQUENCES: 20

(iv) CORRESPONDENCE ADDRESS:
(A) ADDRESSEE: Eric Potter Clarkson
(B) STREET: St Mary's Court, St Mary's Gate
(C) CITY: Nottingham
(D) STATE: Nottinghamshire
(E) COUNTRY: United Kingdom
(F) ZIP: NG1 1LE

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: GB 9123947.5
(B) FILING DATE: 12-NOV-1991

(viii) ATTORNEY/AGENT INFORMATION:
(A) NAME: Bassett, Richard S
(C) REFERENCE/DOCKET NUMBER: IMPF/P9956GB

(ix) TELECOMMUNICATION INFORMATION:
(A) TELEPHONE: +44(0)602 585800
(B) TELEFAX: +44(0)602 588122
(C) TELEX: 37540 Potter G

(2) INFORMATION FOR SEQ ID NO:1:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:1:

GCTGAAGGGC TTTTGAACTC TGCTTA
26

(2) INFORMATION FOR SEQ ID NO:2:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:2:

GCTGAAGGGC TTCTTCCTTA TTGATG
26

(2) INFORMATION FOR SEQ ID NO:3:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:3:

GCTGAAGGGC TTCTGCGTCT CCAT
24

(2) INFORMATION FOR SEQ ID NO:4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:4:

CTGGTCTAAC CAGAGAGACC
20

(2) INFORMATION FOR SEQ ID NO:5:

(i) SEQUENCE CHARACTERISTICS:
(A) LENGTH: 20 base pairs
(B) TYPE: nucleic acid

(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:5:

GCAAGCTTTA TTGAGGCTTA
20

(2) INFORMATION FOR SEQ ID NO:6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:6:

CAGTCAGTCA GTCAGTCAGT
20

(2) INFORMATION FOR SEQ ID NO:7:

(i) SEQUENCE CHARACTERISTICS:
(A) LENGTH: 12 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:7:

CTGCTAGAGA TT
12

(2) INFORMATION FOR SEQ ID NO:8:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:8:

TGCTAGAGAT TTTCCACAC
19

(2) INFORMATION FOR SEQ ID NO:9:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:9:

TTCAAGTCCC TGTTCGGGCG CCAAA
25

(2) INFORMATION FOR SEQ ID NO:10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:10:

GCGTACTCAC CAGTCGCCGC
20

(2) INFORMATION FOR SEQ ID NO:11:

(i) SEQUENCE CHARACTERISTICS:
(A) LENGTH: 14 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:11:

CTGCTAGAGA TTAA
14

(2) INFORMATION FOR SEQ ID NO:12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:12:

ACACCCAATT CTGAAAATGG
20

(2) INFORMATION FOR SEQ ID NO:13:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:13:

TCTCCCTCTT AGCTGGTCCT CTGC
24

(2) INFORMATION FOR SEQ ID NO:14:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:14:

CATGCTTTCA GTGCTCATGG TGTCCTTTC
29

(2) INFORMATION FOR SEQ ID NO:15:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:15:

GATCAGGAAG GAGAAGAGGC TGAGGAACAA
30

(2) INFORMATION FOR SEQ ID NO:16:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:16:

CTCAGCTTGA GGGTTTGC
18

(2) INFORMATION FOR SEQ ID NO:17:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:17:

TTCGTCCTCC TCACAGGGC

19

(2) INFORMATION FOR SEQ ID NO:18:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: YES

(xi) SEQUENCE DESCRIPTION: SEQ ID NO:18:

CACAAGGCCG CCAGCTC
17

(2) INFORMATION FOR SEQ ID NO:19:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO:19:

GAGCTGGCGG CCTTGTG
17

(2) INFORMATION FOR SEQ ID NO:20:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: NO

(iv) ANTI-SENSE: NO

(xi) SEQUENCE DESCRIPTION: SEQ ID NO:20:

CTGGCACGAC GCACACC
17